# EUROPEAN PATENT SPECIFICATION

(11) **EP 2 051 667 B1**
(45) Date of publication and mention of the grant of the patent: **07.09.2011**
(21) Application number: 07766386.2
(22) Date of filing: 30.07.2007
(51) Int. Cl.: A61F 2/40

(54) **SHOULDER REPLACEMENT**
SCHULTERERSATZ
REMPLACEMENT D'ÉPAULE

(30) Priority: 14.08.2006 GB 0616134
(43) Date of publication of application: 29.04.2009
(73) Proprietor: Borowski, Keith, Weavering Maidstone Kent ME14 5UZ (GB)
(72) Inventor: Borowski, Keith, Weavering Maidstone Kent ME14 5UZ (GB)
(74) Representative: Ertl, Nicholas Justin
(86) International application number: PCT/GB2007/002886
(87) International publication number: WO 2008/020161

(56) References cited:
- EP-A- 1 269 941
- FR-A- 2 880 793
- US-A- 3 979 778
- US-A1- 2004 230 311
- US-A1- 2005 177 241

## Description

### Field of the invention

This invention relates to a shoulder replacement prosthesis.

### Background of the invention

The invention is particularly concerned with shoulder replacement carried out following a shoulder fracture although its use may be extended to any condition requiring shoulder replacement. In the case of shoulder fracture injuries, a hemi-arthroplasty is typically carried out to replace only the ball portion of the humeral head.

A conventional prosthesis comprises a head and a stem, and the head of the prosthesis corresponds in shape to the humeral head, in particular the cartilage surface of the humeral head. The stem of the prosthesis is inserted into the top of the humerus shaft, and it is known to provide connection points at the top of the prosthesis stem which enable the greater and lesser tuberosities to be secured to the prosthesis.

The tuberosities are conventionally secured using sutures which are connected to the interface between the tuberosity bone (which has been fractured and come away from the humeral head) and the attached tendon.

There are a number of different ways to secure the tuberosities to the prosthesis. Generally, these methods use a combination of cerclage connections (in which a suture coupled to the tuberosity is secured around or to the shaft of the prosthesis) and longitudinal connections (in which a suture extends down the humerus from the tuberosity at the humerus head to a connection point on the humeral shaft).

A problem with the known shoulder replacement prostheses is that the positioning of the muscles and tendons in the replacement shoulder does not replicate the anatomical configuration. Furthermore, movement of the tuberosities is often not sufficiently inhibited, so that the tendon to joint connection is no longer firm enough to provide a good range of movement and strength. Migration of the tuberosities can also occur. The result of these problems is pain or discomfort to the patient and/or a lack of mobility in the joint. The invention aims to provide an improved shoulder replacement prosthesis which can more reliably replicate the anatomical structure.

US 3 979 778 and US 2005/0177241 each discloses a shoulder prosthesis comprising a spherical head and a shaft.

A shoulder prothesis according to the preamble of claim 1 is disclosed in EP 1 269 941.

### Summary of the invention

According to the invention, there is provided a shoulder replacement prosthesis, as claimed in claim 1.

This prosthesis head has a domed portion which corresponds to the cartilage portion of the humerus head, and an additional flange which acts as a securing area for the tuberosities. By dimensioning the flange appropriately, the anatomical positioning of the tendon to bone connections can be more accurately replicated. In particular, the flange acts to bridge the so-called bare area of the humeral head (the space between the cartilage surface and the greater tuberosity). The flange provides a firm immovable surface area to which connections can be made, so that the length of connections can be reduced, and the scope for movement of the tuberosities in the replacement joint is reduced.

The flange preferably comprises at least three connection portions. For example, four connection portions can be used for the three tendons which connect to the greater tuberosity and the one tendon which connects to the lesser tuberosity. A set of connection devices is then provided for each connection portion.

The connection portions preferably comprise three holes; two for a transverse connection band and one more proximally offset hole for a longitudinal connection band.

Thus, in a preferred example, each set of connection devices comprises a first transverse band and a second longitudinal band.

The transverse band is for coupling a tendon and tuberosity to the respective connection portion by passing the band through two points in the tendon and through the two holes in the flange, with the ends of the bands secured on the under surface of the flange. The transverse band is used to attach the tendontuberosity region to the flange surface for the four tendons. This provides the desired angular positioning around the prosthesis head.

The transverse bands are preferably oriented parallel to the circumferential margin of the flange.

The longitudinal band of each of the four connection devices is used to define a cage to prevent longitudinal movement of the tuberosities, or counter the line of pull of the tendons in whatever plane they assume. The longitudinal band is preferably secured under the flange, passes through the third hole of the connection portion, then over the respective transverse band to a clamp situated on the opposite side of the connection shaft to the head.

The clamp avoids the need to make connections to the existing humerus, which may be susceptible to further cracking.

Each second band is thus preferably for extending from the respective connection portion to the clamp over the first band. In this way, the clamp and the four connection bands define a cage volume secured to the flange, but the second bands also perform a holding function to assist in retaining the position of the first bands.

The clamp itself is preferably connected to a second shaft for securing the clamp with respect to the humerus. Whilst the main shaft is for insertion in the humeral marrow canal, the clamp shaft can be secured from outside the humerus, using bolts/screws passing through the humerus and into the main shaft,or directly into the prosthetic shaft above the fractured end of the humeral bony shaft.

Each band may comprise a partridge band.

The connection between the connection shaft and the head is preferably in the lower third of the height of the head. The head of the prosthesis preferably has a hollow portion, and this gives a large volume of space into which a bone graft can be introduced to promote healing.

As mentioned above, the interface between the domed portion and the flange of the prosthesis corresponds to the limit of the cartilage surface. This interface may thus lie in a plane, and substantially define a circle. The flange surface is then positioned away or extends from this circle (or near circle) in such a way as to substantially define a portion of a cylinder, with different lengths around its circumference. The flange may extend not as a true cylinder, but in a manner which replicates the way the tuberosities extend from the circumference of the native humeral head.

The interface between the domed portion and the flange may include a lip, so that the flange is sunk below the end of the domed portion, for example by approximately 2mm-5mm.

### Brief description of the drawings

The invention will now be described in detail with reference to the accompanying drawings, in which:
Figure 1 shows a prosthesis of the invention, with none of the connection straps shown to facilitate an initial explanation; and
Figure 2 shows the prosthesis of the invention with the connection bands.

### Detailed description

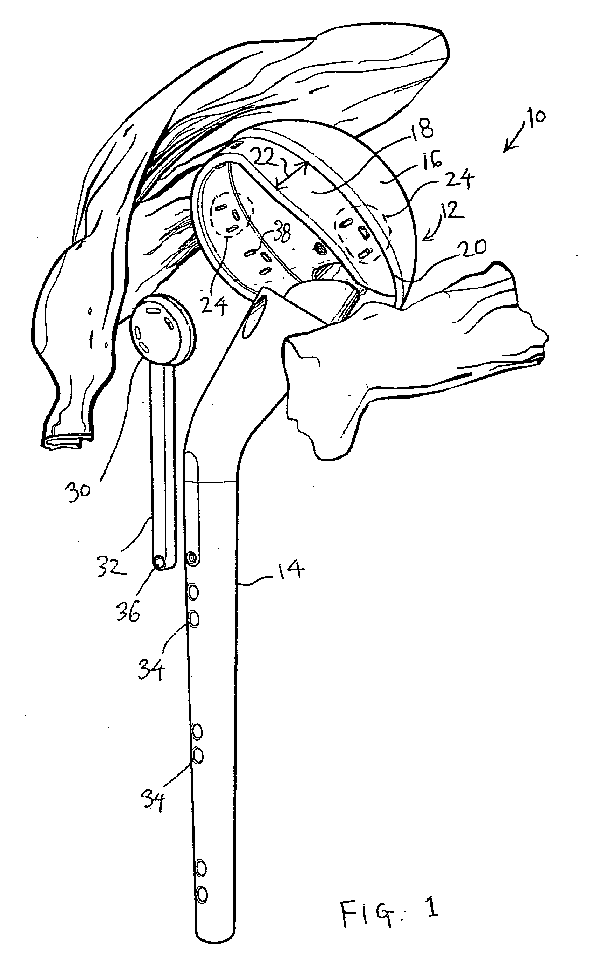

Figure 1 shows the main components of the prosthesis of the invention.

The prosthesis 10 comprises a head 12 for replacing the humeral head, and a connection shaft 14. The head has a domed portion 16 and a flange 18 extending or positioned from an end region 20 of the domed portion.

This end region 20 is the interface between the domed portion 16 and the flange 18, and corresponds to the limit of the cartilage surface of the humerus head. The end region thus defines the anatomical neck of the humeral head. The domed portion 16 corresponds in shape to the head of a conventional shoulder implant prosthesis. The shape of the end region 20 is essentially circular and lies in a plane, so that the domed portion has a shape which is essentially a portion of a sphere defined by dissecting a sphere with a plane.

The flange 18 extends or is positioned from this interface 20 and substantially defines a portion of a quasi-cylinder, which approximates the shape of insertion points of the cuff tendons into the tuberosities and the bare area. This line of insertion veers in parts substantially away from the anatomical neck. Thus, the overall outer shape of the head is of a quasi-cylinder portion which is capped with a domed portion.

The quasi-cylindrical flange 18 has a width 22 (i.e. the local length of the quasi-cylinder) which varies around its circumference. There is also an inward lip between the end of the domed portion and the flange, so that the flange is approximately 4mm recessed compared to the dome margin.

The flange is provided with four connection portions 24, and these are for securing the tendons attached to the tuberosities of the fractured shoulder joint.

The width 22 of the flange 18 varies, so that the flange provides a connection surface which is suitable for the tuberosity tendon to be connected to the flange at each location. Although not shown in the Figures, the positioning of the connection portions 24 along the width of the flange may also be different for the different connection portions.

One of the connection portions is for the lesser tuberosity, where there is one rotator cuff tendon connection to make, and the width of the flange is in the range 5mm to 8mm.

The other three connection portions are for the greater tuberosity, where there are three rotator cuff tendon connections to make, and the width of the flange is in the region of approximately 5mm (in the range 1 mm to 8mm) in the region of the suprapinatus tendon, gradually increasing to approximately to 12mm (in the range 8mm to 25mm) at the teres minor tendon at the back (the left hand side of Figure 1).

Thus, the flange has greater width at a part corresponding to a region of the greater tuberosity than at a part corresponding to a region of the lesser tuberosity.

The flange can increase uniformly in width from its minimum to its maximum (5mm to 12mm for the example dimensions above), and spans approximately 120 to 160 degrees.

The flange design enables the anatomical positioning of the tendon to bone connections to be more accurately replicated. In particular, the flange acts to bridge the so-called bare area of the humeral head so that the tuberosities are connected in more anatomical positions. The flange 18 provides a firm immovable surface area to which connections can be made so that the length of connections can be reduced compared to conventional suturing techniques, and the scope for movement of the tuberosities in the replacement joint is reduced.

The prosthesis also includes a clamp 30, and by coupling the clamp 30 to each of the connection portions 24, a cage can be defined which surrounds the tuberosities, and this can be used to prevent longitudinal movement of the tuberosities or movement in the line of pull of the tendons.

The clamp 30 has a shaft 32 to be secured to the main shaft 14. In use, the main shaft is inserted into the humerus (as is conventional) and secured with cement and/or pins which couple to openings 34 in the main shaft 14. The clamp shaft 32 has an opening 36 (or a plurality of openings), and is for coupling to the humerus and the main shaft 14 using this opening (or openings) 36. The clamp shaft 32 is located on the outside of the humerus in use, and connects through the bone to the main shaft 14 (or it may be smaller and connect directly to the prosthesis above the bony shaft).

As shown in Figure 1, each connection portion 24 has three openings 38. Two of these are to enable a band to be connected around the flange and the tendon in a closed loop, and one of these is to enable a connection to be made between the connection portion 24 and the clamp 30.

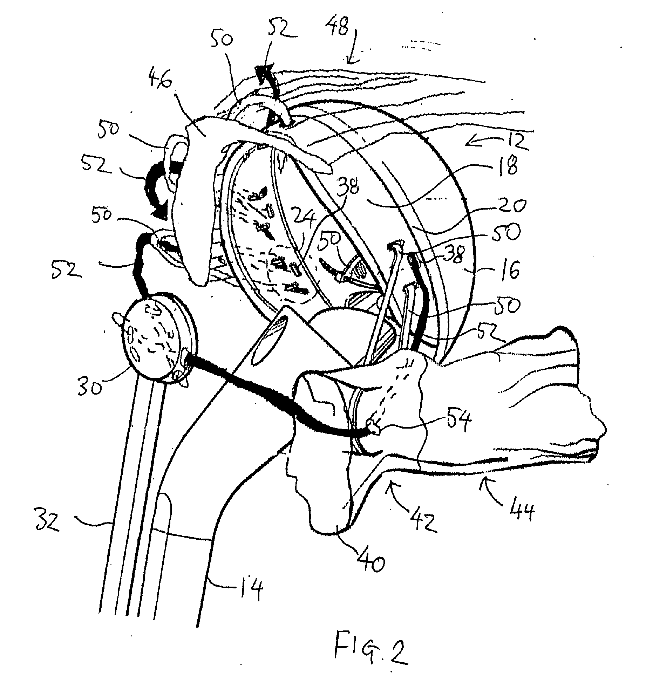

Figure 2 shows the prosthesis with the connection bands. The same reference numerals are used for the same components as in Figure 1.

Figure 2 shows schematically the lesser tuberosity 40 and associated tendon 42 and muscle 44. The greater tuberosity is shown as 46, and the associated muscles and tendons are shown schematically as 48 (shown transparent so that the prosthesis features can be seen).

Each connection portion 24 has two bands, for example partridge bands. These have been used for holding fractured bones together, and comprise a toothed shaft, which engages with a head to be locked in position (similar to a cable tie).

These are easy to install and adjust to the correct length, and have a rectangular cross section. The cross section is suitable for transferring high loads against the broad receptive surface of the flange, and resists tissue tearing. The band cross section also provides some rigidity.

The bands can be modified as necessary so that the surface area used to apply pressure may be ribbed or fine toothed for better grip without strangling the blood supply of the tendon. The ends may be low profile. The locking portions of the bands may be single, i.e. a closed loop, or double, i.e. one at each end of the band in which case the locking portion may abut against the under surface of the flange or clamp as the case may be.

The first band 50 connects around two of the openings 38 in the flange 18 and around the tendon/tuberosity junction. This defines a closed loop which can be tightened to secure the respective tuberosity to the flange 18. One such first band 50 is shown in detail in Figure 2 associated with the lesser tuberosity 40, and the other three are shown only schematically in white.

The second band 52 has a head which seats behind the third opening 38 of the connection portion 24, and again passes through the tuberosity/tendon junction, as shown at 54. It then passes to the clamp 30 where it is secured. With all four second bands 52 connected to the clamp 30, a cage is defined which surrounds the flange 18 and holds the tuberosities in place.

Only two second bands 52 are shown connected to the clamp 30 for simplicity, and the other two are shown with arrow heads to indicate that they also extend to the clamp 30.

The second bands 52 pass over the first band 50, as shown most clearly for the lesser tuberosity 40. In this way, the cage defined by the second bands 52 serves to hold down and reinforce the first bands 50.

The first bands 50 may be considered as transverse connecting bands, and the second bands may be considered as longitudinal connecting bands.

The clamp 30 is situated on the opposite side of the main shaft 14 junction to the head 12. The clamp 30 avoids the need to make suture or band connections to the existing humerus.

In the example shown, the head 12 and shaft 14 of the prosthesis are separate components and can be connected with an interference fit (mortice and taper). This fitting is at the lower part of the head 12, in particular in the lower third of the height of the head. The head of the prosthesis is preferably as hollow as will allow whilst providing the required strength for connection to the shaft, and this gives a large volume of space into which a bone graft can be introduced to promote healing. In particular, the prosthesis has no lateral fin as in conventional designs. The volume beneath the domed head can be used for strut grafts (as mentioned above), but it also makes the surgical procedure easier as there is more space to provide the required sutures or tighten the transverse ties.

The strut grafts mentioned above are inserted after the transverse ties (which attach the tendons to the flange) and before the lateral ties (which connect to the clamp 30 and close the area). The combination of the lateral and transverse ties provide a Mason-Allen type configuration.

The shaft 14 is flattened side to side in the connection region to reduce the presence of metal in the connection areas for the tuberosities, whilst retaining sufficient strength.

In another example, the prosthesis is a single component. Any of the components of the prosthesis may be made modular, e.g. the head separate from the shaft, the head separate from the flange etc.

The prosthesis of the invention is more stable than previous designs in all planes and can be used with weak bones, as the only connection to the humerus is with the main shaft.

The tuberosities are secured with the bands. To assemble the complete prosthesis, the transverse band is passed through two points in the tendon and through the two holes in the flange, with the ends of the bands secured on the under surface of the flange as shown in Figure 2. The longitudinal band is secured under the flange by its head, passes through the third hole of the connection portion, and then passes through the tendon, and over the respective transverse band to the clamp where the other end is secured.

The dome 16 and shaft 14 are metal components, and the bands are plastic or other suitable material. The clamp may be metal and/or plastic or other suitable material.

In the example above, the clamp 30 attaches to the shaft 14 using a clamp shaft 32. This is not essential, and the clamp may screwed directly into an upper part of the shaft 14. This means that the attachment of the clamp 30 is not through bone, and the risk of bone damage (cracks, detached pieces) is reduced. In either case, the method of vertical fixation relies on the stem of the prosthesis, and the connection may or may not pass through the bony shaft, depending on the implementation. If the connection does not need to rely on the bony shaft, this avoids bone cut out.

In the preferred examples, the bands 50,52, are partridge bands, but any other connection devices can be used, such as sutures.

The flange in the prosthesis of the invention not only provides anatomical positioning of the tendon attachment points (by mimicking the rotator cuff) and replaces the bone bare area, but also provides a firm surface against which the tendons can be compressed when attached. This provides greatly improved fixation strength.

In summary, the invention provides a prosthesis in which the attachment points are provided along the line of the anatomical tendinous insertion points of the rotator cuff tendons, thus positioning the tuberosities anatomically.

The attachment points on the flange provide a broad surface area againt which the cuff - tuberosity interface can be compressed by a tie type structure. This is a broader, firmer attachment by which more pressure can be applied, and thus provides a more secure hold and less cut through of the tendon.

The attachment points and ties are arranged to provide a Mason-Allen type configuration offering better tendon-bone purchase.

By designing the stem to connect to the lower part (the lower half or even lower third) of the dome shaped head, the prosthetic shaft replicates the internal medial cortex of the humeral shaft. The resulting absence of a lateral fin means that a very large area is available for bone graft, and a large area is available to work in when fixing the tuberosities. This design removes prosthetic stem insertional metal above the equator of the dome.

The prosthesis has been described as of particular use in the case of shoulder fracture injuries, but the invention can be applied to any situation in which shoulder replacement is required, for example for arthritic patients or other shoulder conditions.

Various modifications will be apparent to those skilled in the art.

## Claims

1. A shoulder replacement prosthesis, comprising a head (12) for replacing the humeral head and a connection shaft (14), wherein the head (12) comprises a domed portion (16) and
a flange (18) having a quasi-cylindrical outer surface positioned at an end region (20) of the domed portion (16), wherein the flange (18) is provided with a plurality of connection portions (24) and associated surface area for securing the tendons attached to the tuberosities, **characterised,**
**in that** the flange (18) has a width (22) parallel to the cylinder axis which varies around the domed portion (16), having a greater width at a part corresponding to a region of the greater tuberosity than at a part corresponding to a region of the lesser tuberosity, such that a rim of the flange (18) away from the domed portion (16) represents the position of the anatomical tendinous insertion points of the rotator cuff on the greater and lesser tuberosities.

2. A prosthesis as claimed in claim 1, wherein the flange (18) comprises at least three connection portions (24).

3. A prosthesis as claimed in claim 2, wherein the flange (18) comprises exactly four connection portions (24).

4. A prosthesis as claimed in claim 2 or 3, further comprising a set of connection devices (50) associated with each connection portion (24).

5. A prosthesis as claimed in claim 4, wherein each set of connection devices (50) comprises a first band (50) for coupling a tendon and tuberosity to the respective connection portion, and a second band (52).

6. A prosthesis as claimed in claim 5, further comprising a clamp (30), wherein each second band (52) is connected between the respective connection portion and the clamp (30), and the clamp is situated on the opposite side of the connection shaft (14) to the head (12).

7. A prosthesis as claimed in claim 6, wherein each second band (52) is for extending from the respective connection portion (24) to the clamp (30) over the first band (50).

8. A prosthesis as claimed in claim 7, further comprising a second shaft (32) connected to the clamp (30) for securing the clamp with respect to the humerus.

9. A prosthesis as claimed in any one of claims 5 to 8, wherein each band (50,52) comprises a partridge band or plastic cable tie device.

10. A prosthesis as claimed in any preceding claim, wherein the width of the flange (18) is in the region of 1 mm to 8mm in the lesser tuberosity region and up to a maximum of between 8mm and 25mm in the greater tuberosity region.

11. A prosthesis as claimed in any preceding claim, wherein the connection between the connection shaft (14) and the head (12) is in the lower third of the height of the head.

12. A prosthesis as claimed in any preceding claim, wherein the flange (18) extends from the end region of the domed portion.

13. A prosthesis as claimed in any preceding claim wherein the interface between the domed portion (16) and the flange (18) lies in a plane, and substantially defines a circle.

14. A prosthesis as claimed in claim 1, wherein the flange (18) is sunk from the end margin of the dome by a lip.

## Patentansprüche

1. Schulterersatzprothese, die einen Kopf (12) zum Ersetzen des Humeruskopfes und einen Verbindungsschaft (14) umfasst, wobei der Kopf (12) einen Kuppelabschnitt (16) aufweist, und
einen Flansch (18) mit einer quasi-zylindrischen Außenfläche, die sich an einer Endregion (20) des Kuppelabschnitts (16) befindet, wobei der Flansch (18) mit mehreren Verbindungsabschnitten (24) und einem assoziierten Oberflächenbereich zum Befestigen der an den Tuberositas angebrachten Sehnen versehen ist, **dadurch gekennzeichnet, dass**
der Flansch (18) eine Breite (22) parallel zur Zylinderachse hat, die um den Kuppelabschnitt (16) herum variiert, wobei die an einem Teil, der einer Region mit größerer Tuberosität entspricht, größer ist als an einem Teil, der einer Region mit geringerer Tuberosität entspricht, so dass ein Rand des Flansches (18) fern vom Kuppelabschnitt (16) die Position der anatomischen Sehneneinsetzpunkte der Rotatormanschette an den größeren und kleineren Tuberositas repräsentiert.

2. Prothese nach Anspruch 1, wobei der Flansch (18) wenigstens drei Verbindungsabschnitte (24) umfasst.

3. Prothese nach Anspruch 2, wobei der Flansch (18) genau vier Verbindungsabschnitte (24) umfasst.

4. Prothese nach Anspruch 2 oder 3, die ferner einen Satz von mit jedem Verbindungsabschnitt (24) assoziierten Verbindungsvorrichtungen (50) umfasst.

5. Prothese nach Anspruch 4, wobei jeder Satz von Verbindungsvorrichtungen (50) ein erstes Band (50) zum Koppeln einer Sehne und Tuberosität mit dem jeweiligen Verbindungsabschnitt und ein zweites Band (52) umfasst.

6. Prothese nach Anspruch 5, die ferner eine Klammer (30) umfasst, wobei jedes zweite Band (52) zwischen dem jeweiligen Verbindungsabschnitt und der Klammer (30) verbunden ist und die Klammer sich auf der gegenüberliegenden Seite des Verbindungsschafts (14) und dem Kopf (12) befindet.

7. Prothese nach Anspruch 6, wobei jedes zweite Band (52) so ausgelegt ist, dass es vom jeweiligen Verbindungsabschnitt (24) zur Klammer (30) über das erste Band (50) verläuft.

8. Prothese nach Anspruch 7, die ferner einen zweiten Schaft (32) umfasst, der mit der Klammer (30) verbunden ist, um die Klammer mit Bezug auf den Humerus zu befestigen.

9. Prothese nach einem der Ansprüche 5 bis 8, wobei jedes Band (50, 52) ein Partridge-Band oder eine Kabelbindervorrichtung aus Plastik umfasst.

10. Prothese nach einem der vorherigen Ansprüche, wobei die Breite des Flansches (18) im Bereich von 1 mm bis 8 mm in der Region mit kleinerer Tuberosität und bis zu maximal 8 mm bis 25 mm in der Region mit größerer Tuberosität liegt.

11. Prothese nach einem der vorherigen Ansprüche, wobei die Verbindung zwischen dem Verbindungsschaft (14) und dem Kopf (12) im unteren Drittel der Höhe des Kopfes ist.

12. Prothese nach einem der vorherigen Ansprüche, wobei sich der Flansch (18) von der Endregion des Kuppelabschnitts erstreckt.

13. Prothese nach einem der vorherigen Ansprüche, wobei die Grenzfläche zwischen Kuppelabschnitt (16) und Flansch (18) in einer Ebene liegt und im Wesentlichen einen Kreis definiert.

14. Prothese nach Anspruch 1, wobei der Flansch (18) vom Endrand der Kuppel durch eine Lippe versenkt wird.

## Revendications

1. Prothèse de remplacement d'épaule, comprenant une tête (12) pour remplacer la tête humérale et une tige de connexion (14), dans laquelle la tête (12) comprend une portion en dôme (16) et une bride (18) ayant une surface externe quasi cylindrique positionnée au niveau d'une région d'extrémité (20) de la portion en dôme (16), dans laquelle la bride (18) est pourvue d'une pluralité de portions de connexion (24) et d'une aire associée pour fixer les tendons reliés aux tubérosités, **caractérisée en ce que** la bride (18) possède une largeur (22) parallèle à l'axe cylindrique qui varie autour de la portion en dôme (16) ayant une largeur supérieure au niveau d'une partie correspondant à une région du trochiter qu'au niveau d'une partie correspondant à une région du trochin, de sorte qu'un bord de la bride (18) à l'écart de la portion en dôme (16) représente la position des points d'insertion tendineux anatomiques de la coiffe des rotateurs sur le trochiter et le trochin.

2. Prothèse selon la revendication 1, dans laquelle la bride (18) comprend au moins trois portions de connexion (24).

3. Prothèse selon la revendication 2, dans laquelle la bride (18) comprend exactement quatre portions de connexion (24).

4. Prothèse selon la revendication 2 ou 3, comprenant en outre un ensemble de dispositifs de connexion (50) associés à chaque portion de connexion (24).

5. Prothèse selon la revendication 4, dans laquelle chaque ensemble de dispositifs de connexion (50) comprend une première bandelette (50) pour raccorder un tendon et une tubérosité à la portion de connexion respective, et une seconde bandelette (52).

6. Prothèse selon la revendication 5, comprenant en outre un clamp (30), dans laquelle chaque seconde bandelette (52) est connectée entre la portion de connexion respective et le clamp (30), et le clamp est situé sur le côté opposé de la tige de connexion (14) à la tête (12).

7. Prothèse selon la revendication 6, dans laquelle chaque seconde bandelette (52) est pour l'extension depuis la portion de connexion respective (24) vers le clamp (30) sur la première bandelette (50).

8. Prothèse selon la revendication 7, comprenant en outre une seconde tige (32) connectée au clamp (30) pour fixer le clamp par rapport à l'humérus.

9. Prothèse selon l'une quelconque des revendications 5 à 8, dans laquelle chaque bandelette (50, 52) comprend une bandelette Partridge ou un dispositif de collier de serrage plastique.

10. Prothèse selon l'une quelconque des revendications précédentes, dans laquelle la largeur de la bride (18) est dans la région de 1 mm à 8 mm dans la région du trochin et jusqu'à un maximum compris entre 8 mm et 25 mm dans la région du trochiter.

11. Prothèse selon l'une quelconque des revendications précédentes, dans laquelle la connexion entre la tige de connexion (14) et la tête (12) est dans le tiers inférieur de la hauteur de la tête.

12. Prothèse selon l'une quelconque des revendications précédentes, dans laquelle la bride (18) s'étend depuis la région d'extrémité de la portion en dôme.

13. Prothèse selon l'une quelconque des revendications précédentes, dans laquelle l'interface entre la portion en dôme (16) et la bride (18) se situe dans un plan et définit essentiellement un cercle.

14. Prothèse selon la revendication 1, dans laquelle la bride (18) est abaissée depuis la marge d'extrémité du dôme par une lèvre.
